# EUROPÄISCHE PATENTSCHRIFT

(11) **EP 0 705 815 B1**
(45) Veröffentlichungstag und Bekanntmachung des Hinweises auf die Patenterteilung: **14.01.1998**
(21) Anmeldenummer: 95113358.6
(22) Anmeldetag: 25.08.1995
(51) Int. Cl.: C07C 227/10, C07C 229/30, C07D 215/56

(54) **Verfahren zur Herstellung von N-Arylaminoacrylsäurederivaten**
Process for the preparation of N-arylaminoacrylic acid derivatives
Procédé pour la préparation de dérivés de l'acide N-arylamino-acrylique

(30) Priorität: 07.09.1994 DE 4431821
(43) Veröffentlichungstag der Anmeldung: 10.04.1996
(73) Patentinhaber: BAYER AG, 51368 Leverkusen (DE)
(72) Erfinder: Rauchschwalbe, Günter, Dr., D-51375 Leverkusen (DE); Beitzke, Bernhard, Dr., D-51503 Rösrath (DE); Eymann, Wolfgang, Dr., D-51061 Köln (DE); Fiege, Helmut, Dr., D-51373 Leverkusen (DE)

(56) Entgegenhaltungen:
- EP-A- 0 388 744
- EP-A- 0 457 090
- EP-A- 0 504 693
- EP-A- 0 608 725
- JP-A- 9 085 255
- US-A- 4 994 599
- Acta Chim. Acad. Scient. Hung. 74(3) (1972) 351-356 (Egri et al.)
- Acta Chim. Acad. Scient. Hung. 78(2) (1973) 217-225 (Egri et al.)
- J. Heteroc. Chem. 17 (1980) 1729-1731 (Coppola et al.)
- J. Heteroc. Chem. 24 (1987) 1537-1539 (Chu et al.)

## Beschreibung

Die vorliegende Erfindung betrifft zunächst ein vorteilhaftes Verfahren zur Herstellung von sonst nur auf unbefriedigende Weise zugänglichen N-Arylaminoacrylsäurederivaten.

Es ist bekannt N-Arylaminoacrylsäurederivate herzustellen, indem man Benzoylessigsäureester mit Trialkylorthoformiat zu β-Alkoxyacrylsäureesterderivaten kondensiert und diese dann mit N-Arylaminen umsetzt (s. J. Med. Chem. 28, 1558-1564 (1985)). Dabei muß die Kondensation mit Trialkylorthoformiat in überschüssigem Acetanhydrid durchgeführt werden. Dieses Acetanhydrid muß vor dem Zusatz von N-Arylamin vollständig abgetrennt werden, weil sonst N-Arylamin mit Acetanhydrid reagiert. Eine Abwesenheit von Acetanhydrid bei der Kondensation mit Trialkylorthoformiat führt zu einer drastischen Verminderung der Ausbeute (s. J. Am. Chem. Soc. 74, 4889 (1952)). Die Reaktionstemperatur liegt bei 130°C oder höher. Dabei bilden sich stark färbende Nebenkomponenten unbekannter Struktur, die nur schwierig abgetrennt werden können.

Außerdem kann im zweiten Verfahrensschritt das zugesetzte N-Arylamin nicht nur in der gewünschten Position reagieren, sondern auch an anderen aktivierten Positionen, z.B. am Arylkern (in p-Position zur Carbonylgruppe) oder an der Esterfunktion (unter Amid-Bildung). Diese Nebenreaktionen finden zwar nur in untergeordnetem Maße statt, trotzdem sind sie höchst unerwünscht, weil sie zu einem Ausbeuteverlust führen und zusätzlichen Trennaufwand erfordern. N-Arylaminoacrylsäurederivate sind nämlich Zwischenprodukte zur Herstellung von Pharmazeutika und müssen deshalb in größtmöglicher Reinheit bereitgestellt werden.

Die Herstellung von N-Arylaminoacrylsäurederivaten über Iminoether ist nur auf einem Umweg bekannt (s. EP-A1 565 132 und J. Med. Chem. 36, 1580 (1993)). Dabei werden (substituierte) Benzoylessigester mit N,N-Dialkylformamidacetal zu Dialkylenaminen umgesetzt, die dann in einer zweiten Reaktionsstufe mit N-Arylamin zum gewünschten Produkt führen. Dabei wird N,N-Dialkylamin freigesetzt, das aus ökologischen Gründen mit großem Aufwand abgetrennt und beseitigt werden muß. Außerdem neigen N,N-Dialkylamine dazu, unter den Bildungsbedingungen mit Halogenatomen an aromatischen Ringen zu reagieren. Dabei entstehen unerwünschte Nebenprodukte, die aus den obengenannten Gründen mit erheblichem Aufwand abgetrennt werden müssen.

Bestimmte N-Arylaminoacrylsäurederivate können auch erhalten werden aus N-unsubstituierten Chinoloncarbonsäuren durch Umsetzung mit 4-Nitrofluorbenzol (Arylierung), Reduktion und Fluorierung nach Balz-Schiemann (s. Collect. Czech. Chem. Commun. 54 2181 (1989)). Die Gesamtausbeute dieses vielstufigen Verfahrens ist niedrig (z.B. 61 % bei der Arylierung), und bestimmte Substitutionsmuster sind auf diese Weise nicht zugänglich.

Es wurde nun ein Verfahren zur Herstellung von N-Arylaminoacrylsäurederivaten der Formel (I) gefunden 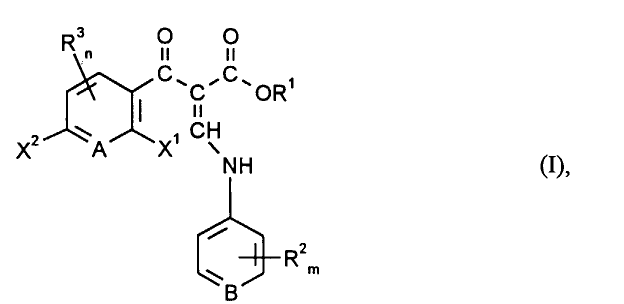 in der
- R¹: Wasserstoff oder C₁-C₆-Alkyl,
- R² und R³: unabhängig voneinander jeweils Fluor, Chlor, Brom, Trifluormethyl, Cyano, Nitro, C₁-C₆-Alkoxy, Hydroxy, Imidazolyl, Triazolyl oder C₇-C₁₀-Aralkoxy oder
- R² und R³: gemeinsam einen -O-, -CH₂-, -CH₂O-, -O-CH₂-O- oder -O-CH₂-CH₂- O-Rest,
- X¹ und X²: unabhängig voneinander jeweils Fluor, Chlor oder Brom,
- A: CH, N oder CR³ mit R³ = Fluor, Chlor, Brom, Trifluormethyl, Cyano, Nitro, C₁-C₆-Alkoxy, Hydroxyl, Imidazolyl, Triazolyl oder C₇-C₁₀-Aralkoxy,
- B: CH, N oder CR² mit R² = Fluor, Chlor, Brom, Trifluormethyl, Cyano, Nitro, C₁-C₆-Alkoxy, Hydroxyl, Imidazolyl, Triazolyl oder C₇-C₁₀-Aralkoxy,
- m: Null, 1, 2 oder 3 und
- n: Null, 1 oder 2 bedeuten,
das dadurch gekennzeichnet ist, daß man ein Benzoylessigsäurederivat der Formel 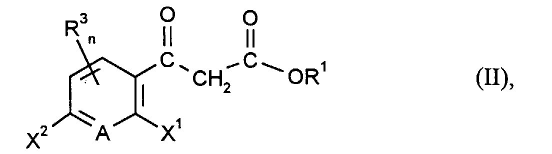 in der
R¹, R³, X¹, X², A und n die bei Formel (I) angegebene Bedeutung haben,
mit einem N-Aryliminoether der Formel 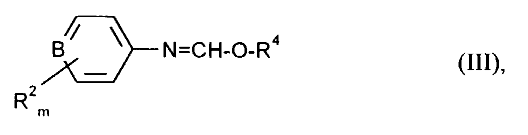 in der
- R², B und m: die bei Formel (I) angegebene Bedeutung haben und
- R⁴: für C₁-C₆-Alkyl steht,
bei 50 bis 120°C in einer Reaktionszeit von 1 bis 10 Stunden
umsetzt.

In den Formeln (I) und (II) stehen
- R¹: vorzugsweise für C₁-C₄-Alkyl,
- R³: vorzugsweise für Fluor oder Chlor,
- X¹ und X²: unabhängig voneinander vorzugsweise jeweils für Fluor oder Chlor,
- A: vorzugsweise für CH oder CR³ mit R³ = Fluor oder Chlor,
- B: vorzugsweise für CH, N oder CR² mit R² = F, Cl, Br oder Nitro,
- n: für Null oder 1.

In den Formeln (I) und (III) stehen
- R²: vorzugsweise für Fluor und
- m: für Null oder 1.

In Formel (III) steht R⁴ vorzugsweise für C₁-C₄-Alkyl.

Die Ausgangsprodukte für das erfindungsgemäße Verfahren sind bekannt. Benzoylessigsäurederivate der Formel (II) und ihre Herstellung sind z.B. beschrieben in Synthesis 1993, 290 und in US-PS 5 262 559. Besonders bevorzugt einzusetzende Benzoylessigsäurederivate der Formel (II) sind 2,3,4,5-Tetrafluorbenzoylessigsäuremethylester, 2,4,5-Trifluorbenzoylessigsäuremethylester, 3-Chlor-2,4,5-trifluorbenzoylessigsäuremethylester, 2,4-Dichlor-5-fluorbenzoylessigsäuremethylester, 4-Nitro-2-chlorbenzoylessigsäuremethylester, 2,6-Dichlor-5-fluor-3-nicotinoylessigsäuremethylester, 5,6-Dibrom-3,4-dioxolo-benzoylessigsäuremethylester und 4-Brom-2-chlorbenzoylessigsäureethylester sowie die entsprechenden Ethyl- bzw. Methylester.

N-Aryliminoether der Formel (III) sind z.B. beschrieben in DE-A 17 68 004, C.A. 64, 2024 und C.A. 84, 179 844. Besonders bevorzugt werden N-Aryliminoether der Formel (III) eingesetzt, bei denen R⁴ für Methyl oder Ethyl steht, die Gruppe R²ₘ für 2-Fluor, 2-Chlor, 3-Trifluormethyl, 2-Trifluormethyl oder 3,4-Methylendioxy steht und B für CH, N oder CR² mit R² = F, Cl, Br oder Nitro steht.

Das erfindungsgemäße Verfahren wird bei Temperaturen im Bereich von 50 bis 120°C, insbesondere von 70 bis 100°C durchgeführt.

Man kann das erfindungsgemäße Verfahren ohne Lösungsmittel durchführen. Zweckmäßigerweise wählt man dann die Temperatur mindestens so hoch, daß das Reaktionsgemisch eine Schmelze bildet. Man kann auch in Gegenwart inerter Lösungsmittel arbeiten, beispielsweise in Gegenwart von Toluol, Chlorbenzol, Dichlorbenzol, Oligoethylenglykolmonoalkylether, Oligoethylenglykoldialkylether, Dimethylformamid oder N-Methylpyrrolidon. Bevorzugte Lösungsmittel sind Diethylenglykolmonoalkylether und Diethylenglykoldialkylether. Besonders bevorzugt werden Butoxyethanol und Diglyme.

Benzoylessigsäurederivate der Formel (II) und N-Aryliminoether der Formel (III) können beispielsweise in Molverhältnissen von 0,6 bis 1,2:1 eingesetzt werden. Vorzugsweise beträgt dieses Verhältnis von 0,8 bis 1,05:1.

Zur Beschleunigung der Reaktion und um einen vollständigen Umsatz zu erhalten, ist es vorteilhaft, den gebildeten Alkohol während der Reaktion aus dem Reaktionsgemisch zu entfernen, beispielsweise durch Destillation, gegebenenfalls unter vermindertem Druck.

Das erfindungsgemäße Verfahren kann man gewünschtenfalls durch Zusatz einer kleinen Menge einer starken Säure katalysieren. Geeignet ist beispielsweise Trifluormethansulfonsäure.

Die Reaktionsdauer für das erfindungsgemäße Verfahren beträgt zwischen 1 bis 10 Stunden. Vorzugsweise liegt die Reaktionszeit im Bereich von 2 bis 6 Stunden.

Nach der Durchführung des erfindungsgemäßen Verfahrens liegt das hergestellte N-Arylaminoacrylsäurederivat der Formel (I) in hoher Reinheit vor, meist über 97 %ig. Insbesondere enthält es keine färbenden Nebenprodukte, sondern häufig nur geringe Mengen der eingesetzten Ausgangsprodukte und gegebenenfalls Reste des abgespaltenen Alkohols. In den meisten Fällen kann man das erfindungsgemäß hergestellte N-Arylaminoacrylsäurederivat der Formel (I) ohne weitere Reinigung direkt weiterverwenden. Dies gilt auch, wenn man in Gegenwart eines Lösungsmittels gearbeitet hat. Man kann dann das lösungsmittelhaltige Produkt direkt weiterverwenden.

Falls eine weitere Reinigung gewünscht wird, kann man diese z.B. durch Umkristallisieren vornehmen, z.B. aus Gemischen aus niederen Alkoholen und Wasser. Wenn man in einem Lösungsmittel gearbeitet hat, z.B. in Diglyme, kann man dieses beispielsweise durch Abdestillieren bei vermindertem Druck oder durch Versetzen mit Wasser und Abfiltration des Produktes abtrennen.

Erfindungsgemäß hergestellte N-Arylaminoacrylsäurederivate der Formel (I) eignen sich besonders gut zur Herstellung von 4-Chinolon-3-carbonsäurederivaten der Formel 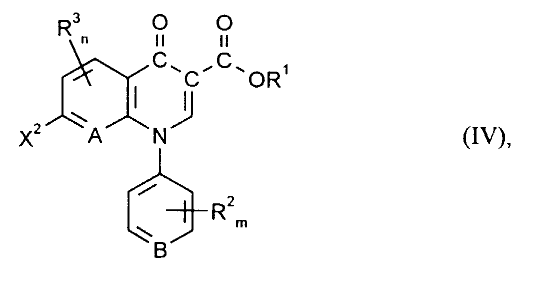 in der
- R¹, R², R³, X², A, B, m und n: die bei Formel (I) angegebene Bedeutung haben,
durch Zusatz einer Hilfsbase in Gegenwart eines Lösungsmittels.

Als Hilfsbase kommen z.B. Magnesium- und/oder Calciumoxid in Frage, als Lösungsmittel z.B. diejenigen, die gegebenenfalls beim erfindungsgemäßen Verfahren eingesetzt werden können.

Die Herstellung der Chinoloncarbonsäurederivate der Formel (IV) aus den N-Arylaminoacrylsäurederivaten der Formel (I) kann beispielsweise bei Temperaturen zwischen 90 und 130°C, vorzugsweise zwischen 100 und 120°C, erfolgen. Für diese Reaktion können als Lösungsmittel beispielsweise protische oder aprotische Lösungsmittel eingesetzt werden, bevorzugt sind Toluol, Chlorbenzol, Dichlorbenzol, Oligoethylenglykol-mono- oder -dialkylether, Dimethylformamid und N-Methylpyrrolidon. Bevorzugt sind polare Lösungsmittel wie Diethylenglykolmono- oder -dialkylether, ganz besonders bevorzugt Butoxyethanol, Diethylenglykoldimethylether und Diethylenglykoldiethylether. Die Lösungsmittel können beispielsweise in einer Menge von 400 bis 1000 ml pro Mol der Verbindung der Formel (I) eingesetzt werden. Vorzugsweise liegt diese Menge bei 500 bis 600 ml pro Mol.

Die Hilfsbasen kann man beispielsweise in Mengen von 0,5 bis 3 Mol pro Mol der Verbindung (I) einsetzen, und vorzugsweise liegt diese Menge bei 1 bis 2 Mol.

Die Aufarbeitung der Ansätze zur Herstellung von Chinoloncarbonsäurederivaten der Formel (IV) kann beispielsweise so erfolgen, daß man Wasser und Säure zugibt, durch Erhitzen restliche Esterfunktionen zur Carbonsäure verseift, anorganische Bestandteile auflöst und das gewünschte Produkt aus der flüssigen Phase durch Filtration abtrennt. Als Säuren kommen beispielsweise Essigsäure, Zitronensäure, Schwefelsäure oder Salzsäure in Frage, bei den anorganischen Bestandteilen kann es sich beispielsweise um restliche Hilfsbase handeln.

Es ist besonders bevorzugt, für diese Verwendung N-Arylaminoacrylsäurederivate der Formel (I) nicht zu isolieren, sondern im gleichen Reaktionsgefäß wie zu deren Herstellung die Umsetzung zu 4-Chinolon-3-carbonsäurederivaten der Formel (IV) vorzunehmen (= Eintopfverfahren). 4-Chinolon-3-carbonsäurederivate der Formel (IV) lassen sich mit geeigneten Aminen zu sehr wirksamen Antibiotika umsetzen (s. US-PS 4 994 599).

Es ist ausgesprochen überraschend, daß auf die erfindungsgemäße Weise Benzoylessigsäurederivate der Formel (II) und N-Aryliminoether der Formel (III) unter milden Bedingungen zu N-Arylaminoacrylsäurederivaten der Formel (I) kondensiert werden können und das noch in sehr hohen Ausbeuten und Reinheiten.

So reagiert z.B. der 2,4-Dichlor-5-fluorbenzoylessigsäuremethylester mit O-Ethyl-N(4-fluorphenyl)-formiminoether ohne Lösungsmittel schon bei 50°C innerhalb von wenigen Stunden und in einem mäßig polaren Lösungsmittel bei 50°C innerhalb von 3 Stunden praktisch vollständig ab. Die Reinheit der hergestellten N-Arylaminoacrylsäurederivate der Formel (I) ist so hoch, daß die Produkte ohne Zwischenisolierung und Reinigung weiterverarbeitet werden können, beispielsweise zu 4-Chinolon-3-carbonsäurederivaten der Formel (IV), die unmittelbar pharmazeutische Qualitätsanforderungen erfüllen. Bei der Herstellung solcher 4-Chinolon-3-carbonsäurederivate erreicht man im allgemeinen Ausbeuten von ca. 85 % der Theorie, bezogen auf eingesetzte N-Aryliminoether der Formel (III).

Das ist umso erstaunlicher, als erwartet werden mußte, daß N-Aryliminoether weniger reaktiv sein würden als N-Alkyliminoether, so daß drastischere Bedingungen als in bekannten Synthesen mit N-Alkyliminoethern erforderlich sein würden, was zur Bildung größerer Mengen an Nebenprodukten und damit schlechteren Ausbeuten und Reinheiten führen sollte. Ähnliche Synthesen mit N-Alkyliminoethern sind beispielsweise beschrieben in Acta Chimica Academiae Scientiarum Hungarica 74 (3), 351-356 (1972), wo Temperaturen von wenigstens 140°C, Reaktionszeiten von wenigstens 24 Stunden, Ausbeuten von maximal 68 % und die Unmöglichkeit der Herstellung von N-Arylderivaten angegeben sind (für Letzteres siehe loc. cit. 78 (2), 217 und 223) und in J. Heterocyclic Chem. 24, 1537 (1987) sowie in loc. cit. 17, 1729 (1980). In den beiden letztgenannten Schriften werden als Reaktionsbedingungen wenigstens 48 Stunden bei 65°C bzw. 72 Stunden bei 110 bis 115°C und Ausbeuten von 80 bzw. 56 % der Theorie angegeben.

### Beispiele

### Beispiel 1

88 g (0,53 Mol) N-(4-Fluorphenyl)-formimino-ethylether wurden vorgelegt und 132 g 2,4-Dichlor-5-fluorbenzoylessigsäuremethylester (0,50 Mol) hinzugefügt. Es wurde im Verlauf von 90 Minuten auf 100°C erwärmt und dabei bei 200 mbar 22 g Ethanol und wenig des eingesetzten Iminoethers abdestilliert. Es wurden 196 g 3-[N-(4-fluorphenylamino)]-2-(2,4-dichlor-5-fluorbenzoyl)-acrylsäuremethylester als blaßgelbes Produkt erhalten. Es enthielt laut Gaschromatogramm je 1 % Ethanol und eingesetzten Iminoether.

### Beispiel 2

Die Reaktanden wurden in den gleichen Mengen wie in Beispiel 1 zusammengegeben und dann bis zur Auflösung des Benzoylessigsäureesters im offenen Becherglas 2 1/2 Tage bei 40 bis 50°C stehengelassen. Dabei erstarrte die Masse. Es wurde das gleiche Produkt wie in Beispiel 1 in hochreiner Form erhalten (blaßgelb, Schmelzpunkt 105°C).

### Beispiel 3

Es wurde verfahren wie in Beispiel 1. Das erhaltene Produkt wurde in 300 ml Diglyme gelöst, 25 g Magnesiumoxid hinzugefügt und 3 Stunden auf 110°C erhitzt. Danach wurden 150 ml 37 %ige wäßrige Salzsäure zugefügt, 6 Stunden am Rückfluß erhitzt und das Gemisch auf 400 ml Wasser ausgetragen. Der ausgefallene Niederschlag wurde abgesaugt, mit Wasser und Isopropanol gewaschen und getrocknet. Es wurden 122 g N-(4-Fluorphenyl)-6-fluor-7-chlor-1,4-dihydro-4-oxo-chinolon-3-carbonsäure von 99 %iger Reinheit erhalten. Das entspricht einer Ausbeute von 73 % der Theorie, bezogen auf eingesetzten Formiminoether.

### Beispiel 4

132,5 g 2,4-Dichlor-5-fluorbenzoylessigsäuremethylester wurden in 83,5 g 4-Fluorphenylformiminoethylether gelöst, ein Tropfen Trifluormethansulfonsäure hinzugefügt und zunächst für 3 Stunden auf 60°C erhitzt. Dabei erstarrte das Produkt. Danach wurde noch 1 Stunde auf 75°C erhitzt, wobei Verflüssigung eintrat, dann noch 1 Stunde auf 90°C und das gebildete Ethanol abdestilliert. Das erhaltene Produkt wurde in 500 ml Butoxyethanol gelöst und mit 80 g Magnesiumoxid bei 116 bis 120°C cyclisiert. Nach der Aufarbeitung entsprechend Beispiel 3 wurden 144 g des gleichen Produkts wie in Beispiel 3 erhalten. Die Ausbeute betrug 85 % der Theorie, bezogen auf eingesetzten Formiminoether.

### Beispiel 5

9,5 g N-(2,4-Difluorphenyl)-formiminoethylether wurden in 13,5 g 2,4-Dichlor-5-fluor-benzoylessigsäuremethylester gelöst und 3 Tage bei 50 bis 60°C stehengelassen. Es wurden 23 g eines blaßgelben Feststoffs erhalten, der gemäß HPLC-Analyse 98 % rein war. Es enthielt je 1 % der Edukte und wies einen Schmelzpunkt von 91°C auf. Dieses Produkt wurde aus einem Ethanol/Wasser-Gemisch umkristallisiert und so 14 g reiner 3-N-(2,4-Difluorphenyl)-amino-2-(2,4-dichlor-5-fluorbenzoyl)-acrylsäuremethylester mit einem Schmelzpunkt von 91°C erhalten. Das entspricht einer Ausbeute von 70 % der Theorie, bezogen auf eingesetzten Formiminoether.

### Beispiel 6

60 ml Ethylenglykolmonobutylether, 37 g N-(2,4-Difluorphenyl)-formiminoethylether und 50 g 2,4-Dichlor-5-fluor-benzoylessigsäureethylester wurden für 5 Stunden auf 60 bis 65°C erwärmt. Bei 20 mbar wurde Ethanol und etwas Lösungsmittel abgezogen. Dann wurde mit 150 ml Ethylenglykolmonobutylether verdünnt, 12 g Magnesiumoxid zugefügt und 10 Stunden auf 110°C erwärmt. Zur Aufarbeitung wurde das Reaktionsgemisch auf ein Gemisch aus 150 ml Wasser und 50 ml 37 %iger wäßriger Salzsäure gegossen, 5 Stunden zum Sieden erhitzt, mit Wasser verdünnt, abgesaugt, mit Wasser und 2-Propanol gewaschen und so 49,5 g 99 % reine N-(2,4-Difluorphenyl)-6-fluor-7-chlor-1,4-dihydro-4-oxo-chinolon-3-carbonsäure erhalten. Das entspricht 70 % der Theorie, bezogen auf eingesetzten Formiminoether.

### Beispiel 7

88 g N-(2-Fluorphenyl)-formiminoethylether wurden vorgelegt und 132 g 2,4-Dichlor-5-fluorbenzoylessigsäuremethylester zugefügt. Dann wurde für 90 Minuten auf 100°C erwärmt und bei 200 mbar 23 g Ethanol und wenig Iminoether abdestilliert. Es wurden 195 g 3-[N-(2-Fluorphenyl)-amino]-2-(2,4-dichlor-5-fluorbenzoyl)-acrylsäuremethylester als blaßgelbes Produkt erhalten. Es enthielt gemäß Gaschromatogramm je ca. 1 % Ethanol und eingesetzten Iminoether.

## Patentansprüche

1. Verfahren zur Herstellung von N-Arylaminoacrylsäurederivaten der Formel (I) 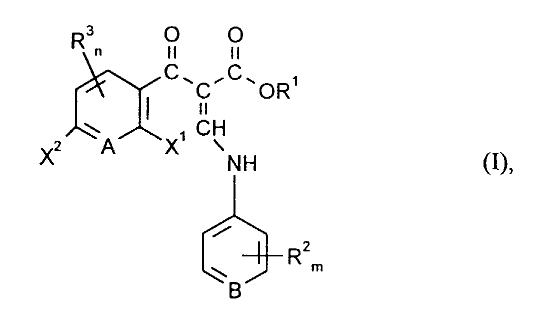 in der
R¹ Wasserstoff oder C₁-C₆-Alkyl,
R² und R³ unabhängig voneinander jeweils Fluor, Chlor, Brom, Trifluormethyl, Cyano, Nitro, C₁-C₆-Alkoxy, Hydroxy, Imidazolyl, Triazolyl oder C₇-C₁₀-Aralkoxy oder
R² und R³ gemeinsam einen -O-, -CH₂-, -CH₂O-, -O-CH₂-O- oder -O-CH₂-CH₂-O-Rest,
X¹ und X² unabhängig voneinander jeweils Fluor, Chlor oder Brom,
A CH, N oder CR³ mit R³ = Fluor, Chlor, Brom, Trifluormethyl, Cyano, Nitro, C₁-C₆-Alkoxy, Hydroxyl, Imidazolyl, Triazolyl oder C₇-C₁₀-Aralkoxy,
B CH, N oder CR² mit R² = Fluor, Chlor, Brom, Trifluormethyl, Cyano, Nitro, C₁-C₆-Alkoxy, Hydroxyl, Imidazolyl, Triazolyl oder C₇-C₁₀-Aralkoxy,
m Null, 1, 2 oder 3 und
n Null, 1 oder 2 bedeuten,
dadurch gekennzeichnet, daß man ein Benzoylessigsäurederivat der Formel 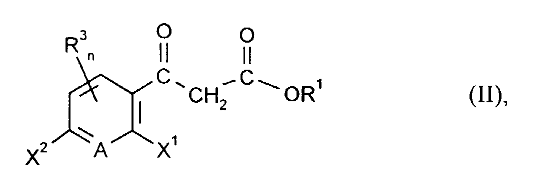 in der
R¹, R³, X¹, X², A und n die bei Formel (I) angegebene Bedeutung haben, mit einem N-Aryliminoether der Formel 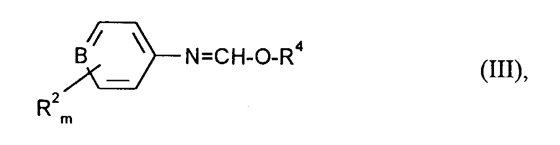
in der
R², B und m die bei Formel (I) angegebene Bedeutung haben und
R⁴ für C₁-C₆-Alkyl steht,
bei 50 bis 120°C in einer Reaktionszeit von 1 bis 10 Stunden umsetzt.

2. Verfahren nach Anspruch 1, dadurch gekennzeichnet, daß in den Formeln (I) und (II)
R¹ für C₁-C₄-Alkyl,
R³ für Fluor oder Chlor,
X¹ und X² unabhängig voneinander jeweils für Fluor oder Chlor,
A für CH oder CR³ mit R³ = Fluor oder Chlor,
B für CH, N oder CR² mit R² = F, Cl, Br oder Nitro,
n für Null oder 1 stehen,
in den Formeln (I) und (III) R² für Fluor
und
m für Null oder 1 stehen und
in Formel (III) R⁴ für C₁-C₄-Alkyl steht.

3. Verfahren nach Ansprüchen 1 bis 2, dadurch gekennzeichnet, daß man es ohne Lösungsmittel durchführt.

4. Verfahren nach Ansprüchen 1 bis 2, dadurch gekennzeichnet, daß man es in Gegenwart von Toluol, Chlorbenzol, Dichlorbenzol, Oligoethylenglykolmonoalkylethern, Oligoethylenglykoldialkylethern, Dimethylformamid oder N-Methylpyrrolidon als Lösungsmittel durchführt.

## Claims

1. Process for the preparation of N-arylaminoacrylic acid derivatives of the formula (I) 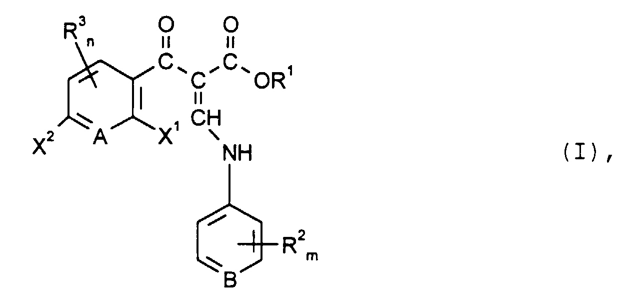 in which
R¹ denotes hydrogen or C₁-C₆-alkyl,
R² and R³ independently of one another each denote fluorine, chlorine, bromine, trifluoromethyl, cyano, nitro, C₁-C₆-alkoxy, hydroxyl, imidazolyl, triazolyl or C₇-C₁₀-aralkoxy or
R² and R³ together denote an -O-, -CH₂-, -CH₂O-, -O- CH-O- or -O-CH₂-CH₂-O- radical,
X¹ and X² independently of one another each denote fluorine, chlorine or bromine,
A denotes CH, N or CR³, where R³ = fluorine, chlorine, bromine, trifluoromethyl, cyano, nitro, C₁-C₆-alkoxy, hydroxyl, imidazolyl, triazolyl or C₇-C₁₀-aralkoxy,
B denotes CH, N or CR², where R² = fluorine, chlorine, bromine, trifluoromethyl, cyano, nitro, C₁-C₆-alkoxy, hydroxyl, imidazolyl, triazolyl or C₁-C₁₀-aralkoxy,
m denotes zero, 1, 2 or 3 and
n denotes zero, 1 or 2,
characterized in that a benzoylacetic acid derivative of the formula 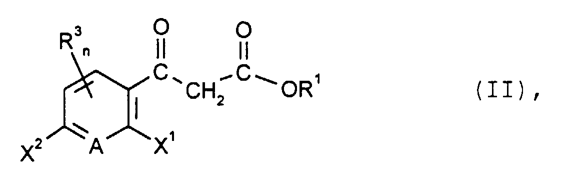 in which
R¹, R³, X¹, X², A and n have the meaning given for formula (I),
is reacted with an N-arylimino ether of the formula 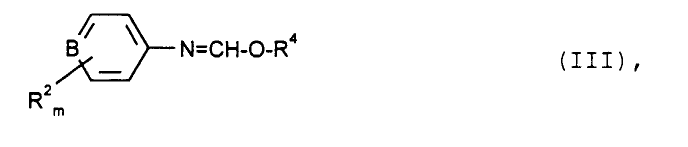 in which
R², B and m have the meaning given for formula (I) and
R⁴ represents C₁-C₆-alkyl,
at 50 to 120°C over a reaction time of 1 to 10 hours.

2. Process according to Claim 1, characterized in that, in the formulae (I) and (II),
R¹ represents C₁-C₄-alkyl,
R³ represents fluorine or chlorine,
X¹ and X² independently of one another each represent fluorine or
chlorine,
A represents CH or CR³, where R³ = fluorine or chlorine,
B represents CH, N or CR², where R² = F, Cl, Br or nitro, and
n represents zero or 1,
in the formulae (I) and (III), R² represents fluorine
and
m represents zero or 1, and,
in formula (III), R⁴ represents C₁-C₄-alkyl.

3. Process according to Claims 1 to 2, characterized in that it is carried out without a solvent.

4. Process according to Claims 1 to 2, characterized in that it is carried out in the presence of toluene, chlorobenzene, dichlorobenzene, oligoethylene glycol monoalkyl ethers, oligoethylene glycol dialkyl ethers, dimethylformamide or N-methylpyrrolidone as a solvent.

## Revendications

1. Procédé de préparation de dérivés d'acide N-arylaminoacrylique de formule (I) 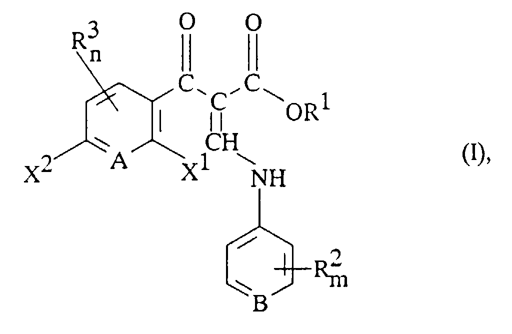 où
R¹ est hydrogène ou un alkyle C₁-C₆,
R² et R³ représentent indépendamment l'un de l'autrc respectivement les fluor, chlore, brome, trifluorométhyle, cyano, nitro, alcoxy C₁-C₆, hydroxyle, imidazolyle, triazolyle ou aralcoxy C₇-C₁₀ ou
R² et R³ représentent ensemble un reste -O-, -CH₂-, -CH₂O-, -O-CH₂-O- ou -O-CH₂-CH₂-O-,
X¹ et X² représentent indépendamment l'un de l'autre respectivement un fluor, chlore ou brome,
A représente CH, N ou CR³ avec R³ = fluor, chlore, brome, trifluorométhyle, cyano, nitro, alcoxy C₁-C₆, hydroxyle, imidazolyle, triazole ou aralcoxy C₇-C₁₀, B représente CH, N ou CR² avec R² = fluor, chlore, brome, trifluorométhyle, cyano, nitro, alcoxy C₁-C₆, hydroxyle, imidazolyle, triazolyle ou araloxy C₇-C₁₀,
m représente 0, 1, 2 ou 3 et
n représente 0, 1 ou 2,
caractérisé en ce qu'on fait réagir un dérivé d'acide benzoylacétique de formule 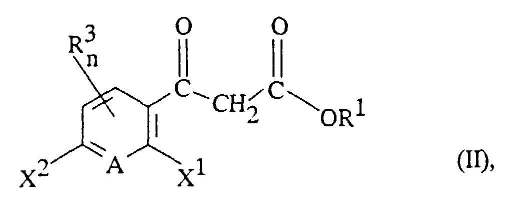 où
R¹, R³, X¹, X², A et n ont la signification donnée pour la formule (I), avec un N-aryliminoéther de formule 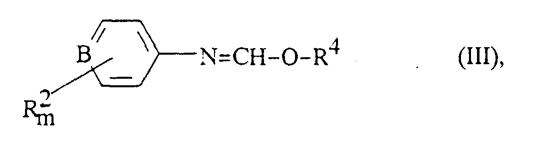 où
R², B et m ont la signification donnée pour la formule (I) et
R⁴ représente un alkyle C₁-C₆,
entre 50 et 120°C pendant une période de réaction de 1 à 10 heures.

2. Procédé selon la revendication 1, caractérisé en ce que dans les formules (I) et (II)
R¹ représente un alkyle C₁-C₄,
R³ représente le fluor ou le chlore,
X¹ et X² représentent indépendamment l'un de l'autre respectivement le fluor ou le chlore,
A représente CH ou CR³ avec R³ = fluor ou chlore,
B représente CH, N ou CR² avec R² = F, Cl, Br ou nitro,
n représente 0 ou 1,
dans les formules (I) et (III) R² représente le fluor
et
m représente 0 ou 1 et
dans la formule (III) R⁴ représente un alkyle C₁-C₄.

3. Procédé selon les revendications 1 à 2, caractérisé en ce qu'on travaille sans solvant.

4. Procédé selon les revendications 1 à 2, caractérisé en ce qu'on travaille en présence de toluène, chlorobenzène, dichlorobenzène, éthers monoalkyliques d'oligoéthylèneglycol, éthers dialkyliques d'oligoéthylèneglycol, diméthylformamide ou N-méthylpyrrolidone comme solvants.
